# EUROPEAN PATENT APPLICATION

(11) **EP 1 224 949 A1**
(43) Date of publication of application: **24.07.2002**
(21) Application number: 00966532.4
(22) Date of filing: 17.10.2000
(51) Int. Cl.: A61N 1/30

(54) **DEVICE AND ELECTRODE FOR ELECTROPORATION**

(30) Priority: 18.10.1999 JP 33175599
(71) Applicant: HISAMITSU PHARMACEUTICAL CO. INC., Tosu-shi, Saga-ken 841-0017 (JP)
(72) Inventor: MORI, Kenji Tsukuba Lab. Hisamitsu Phar. Co., Inc., Tsukuba-shi Ibaraki 305-0856 (JP); KUBO, H. Tsukuba Lab. of Hisamitsu Phar. Co., Inc., Tsukuba-shi Ibaraki 305-0856 (JP); SUGIBAYASHI, Kenji, Kawagoe-shi, Saitama 350-1124 (JP)
(74) Representative: Westendorp, Michael, Dr.
(86) International application number: JP0007188
(87) International publication number: WO0128624

(57) **Abstract**

A device and electrode for electroporation which have a positive electrode and a negative electrode for applying electroporation to skin or mucosa, are provided. The device comprises a preparation (30) having the positive electrode (36) and the negative electrode (37) placed therein and a power supply device (38) for loading a voltage between the positive electrode (36) and the negative electrode (37), in which, by adjusting at least one of the distance between the positive electrode (36) and the negative electrode (37) and a load voltage, in one application of electroporation, the maximum value of the strength of an electric field generated in the surface to which electroporation is applied such as the skin is set to be in the range of about 790 V / cm to 4000 V / cm, and / or the voltage-loaded area average electric field strength is set to be in the range about 7 V / cm to 730 V / cm.

## Description

### Technical Field

The invention relates to a device and electrode for electroporation delivering drugs (physiologically active substance) into a living organism using electroporation.

### Background Art

The electroporation is a method which have been used for introduction of genes, and is used for introducing DNA and the like into a cell by instantaneous applying of a high voltage to the cell.

In recent years, this technology has been applied to transdermal or transmucosal drug delivery (National Publication of International Patent Application No. 1991-502416, Proc. Natl. Acad. Sci., USA, 90, 10504 - 10508, 1993). This describes that by loading a voltage between both positive and negative electrodes of electroporation, a hole is created in skin and mucosa to form a new reversible route, thereby increasing the membrane permeation of materials. Also, Edwards, et. al. (Journal of Controlled Release, 34, 211, 1995 ), A. Jadoul, et. al. (Journal of Controlled Release, 54, 265, 1998), and Rita Vanberver (Journal of Controlled Release, 50, 225, 1998, Journal of Controlled Release, 54, 243, 1998, Pharmaceutical Research, 11, 1657, 1994 ) have reported the effect of electroporation obtained by applying a voltage to skin in the same manner. Also, Hofman, et. al. (Bioelectrochemistry and Bioenrgetic, 38, 209, 1995 ) have succeeded in causing carriers to be absorbed by applying electrodes to skin such that the skin may be sandwiched thereby.

Although these conventional technologies have experimentally obtained the effect of promoting the skin permeation of a drug, electric fields are not considered, or if considered, a load voltage is simply divided by the distance between the positive electrode and the negative electrode. In the example of Hofman, et. al. although an electric field analysis is performed, the electric field is obtained simply by dividing a primarily applied voltage by the thickness of skin corneum immediately underneath the electrodes. Thus, the electric field strength applied to only the skin immediately underneath the electrodes (in the direction of the thickness of skin corneum) is considered, but the electric field strength in the parts not making contact with the electrodes is not considered.

As a result, these methods produce variations in electric fields, such as the possibility that a strong electric field may be produced at a localized portion of skin to damage the skin and, on the other hand, the case in which an electric field is not formed. For this reason, these methods can not be effectively used, and also present a problem of inadequate absorption of a target material into skin.

Therefore, the invention has an object to provide a device and electrode for electroporation which allow more effective skin-absorption of a drug.

### Disclosure of the Invention

In order to resolve the above problems, as a result of performing very hard research, the inventors noted that, in applying an electroporation device to target matter of introduction such as skin or mucosa, the electric field distribution formed in the skin and the like is significantly influenced by parameters such as the position relation between and the shape and area of the positive and negative electrodes making direct or indirect contact with the skin and the like. Thus, the inventors have found that it is effective to set electric field strength so as to optimize the skin absorption of a drug, which are absorbed into bodies.

Therefore, according to the invention, in application of electroporation to target matter of introduction such as skin or mucosa, the maximum electric field value (the maximum value of electric field strength) and / or the voltage-loaded area average electric field strength, obtained from the electric field distribution along the plane (two-dimensional), are specified so as to cause electroporation in the surface to which electroporation is applied and to be in a predetermined range.

That is, according to the invention, in applying electroporation to skin or mucosa, the electric field distribution and the maximum value of the electric field are calculated, and the maximum value of electric field strength and / or volatge-loaded area average electric field strength, generated in the surface to which electroporation is applied of the skin or mucosa, are set to a specific value. Thereby, the invention realizes a device and electrode for use in electroporation which are more excellent in skin permeability.

More specifically, the invention provides an electroporation device in which for each one application of electroporation, the maximum value of the strength of an electric field generated in the surface to which electroporation is applied is set to be in the range of about 790 V / cm to 4000 V / cm.

Also, the invention provides an electroporation device in which for each one application of electroporation, the voltage-loaded area average strength of an electric field generated in the surface to which electroporation is applied is set to be in the range of about 7 V / cm to 730 V / cm.

Further, the invention provides an electroporation device that is configured such that the strength of an electric field generated between the positive electrode and the negative electrode is substantially uniform.

Also, according to the invention, by adjusting at least one of the distance between the positive electrode and the negative electrode and a load voltage, in one application of electroporation, the maximum value of the strength of an electric field generated in the surface to which electroporation is applied is set to be in the range of about 790 V / cm to 4000 V / cm, and / or the voltage-loaded area average electric field strength is set to be in the range of about 7 V / cm to 730 V / cm. Herein, one or both of the positive electrode and the negative electrode may be placed at a distance from the surface to which electroporation is applied, or both may be in contact with the surface to which electroporation is applied.

Further, the invention provides electrode for electroporation in which the electrode structures of the positive electrode and the negative electrode have the same shapes and the distance between the positive electrode and the negative electrode is substantially constant. Herein, the electrode structures of the positive electrode and the negative electrode are preferably of a plate type or of a concentric circle-shaped ring type, respectively. In addition, the opposing planes of the positive electrode and the negative electrode preferably have the shape of projections and depressions with respect to each other. Such electrodes may be applied to the electroporation device.

### Brief Description of the Drawings

Figure 1 is a diagram showing equipotential lines produced in loading electrode for electroporation;
Figure 2 is a diagram showing an electric field distribution obtained by computer software analysis;
Figure 3 is a diagram showing an example of an electoropolation device;
Figure 4 is a perspective diagram showing an example applying a ring type-needle type of electrode to skin;
Figure 5 is a plan view showing an example applying a ring type-needle type of electrode to skin;
Figure 6 is a perspective diagram showing an example applying a plate type-plate type of electrode to skin;
Figure 7 is a plan view showing an example applying a plate type-plate type of electrode to skin;
Figure 8 is a perspective diagram showing an example applying a needle type-needle type of electrode to skin;
Figure 9 is a plan view showing an example applying a needle type-needle type of electrode to skin;
Figure 10 is a graph showing the relation between the maximum value of electric field strength and the maximum permeation rate of sodium benzoate;
Figure 11 is a graph showing the relation between voltage-loaded area average electric field strength and the maximum permeation rate of sodium benzoate;
Figure 12 is a diagram showing an upright permeation experiment diffusion cell;
Figure 13 is a graph showing the relation between voltage-loaded area average electric field strength and the accumulated amount of permeation of diclofenac sodium until the seventh hour;
Figure 14 is a diagram showing the electric field distribution obtained by analyzing the case of a ring type-needle type of electrode by computer software;
Figure 15 is a diagram showing the electric field distribution obtained by analyzing the case of a plate type-plate type of electrode by computer software;
Figure 16 is a diagram showing the electric field distribution obtained by analyzing the case of a needle type-needle type of electrode by computer software;
Figure 17 is a diagram showing an example of electrodes designed based on electric field analyses;
Figure 18 is a diagram showing an example of electrodes designed based on electric field analyses;
Figure 19 is a diagram showing an example of electrodes designed based on electric field analyses;
Figure 20 is a diagram showing an in vitro permeation experiment device and the situation of electrodes mounted therein;
Figure 21 is a diagram showing a three-dimensional distribution of an electric field; and
Figure 22 is a diagram showing the two-dimensional distribution of the electric field in the lowest XY plane of Figure 21.

### Best Mode for Carrying out the Invention

The invention is described taking as an example the phenomena observed in applying electroporation to skin. However, the following descriptions are for illustration, and the invention is not limited to them.

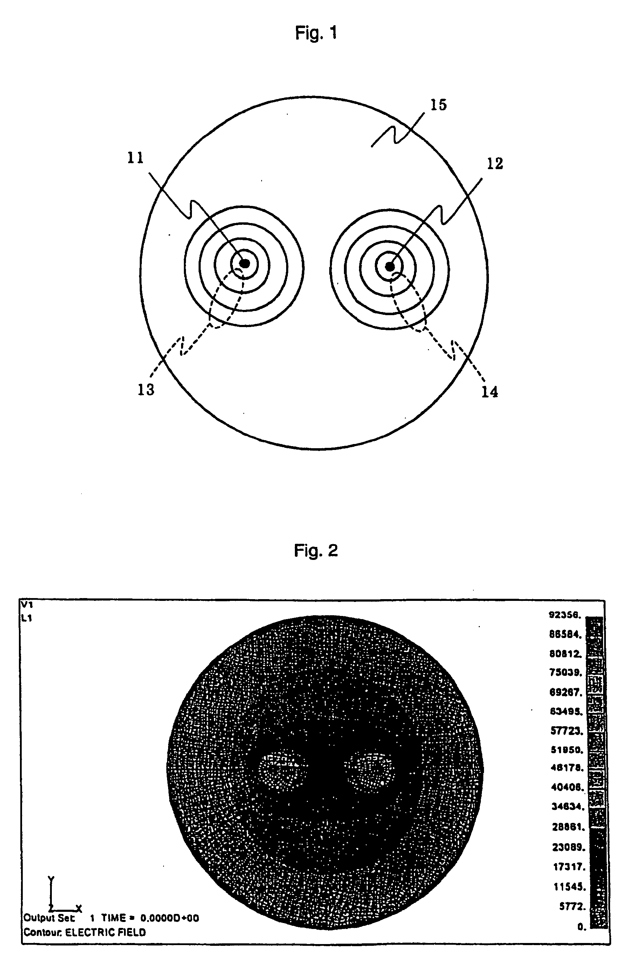

Figure 1 is a diagram showing equipotential lines occurring in loading of an electrode for electroporation. In Figure 1, reference numeral 11 denotes a positive electrode-applied portion, reference numeral 12 denotes a negative electrode-applied portion, reference numerals 13 and 14 denotes equipotential lines, and reference numeral 15 denotes a electrode-applied skin. When a voltage is loaded between the positive electrode-applied portion 11 and the negative electrode-applied portion 12, the circulating equipotential lines 13 and 14 as shown in Figure 1 or ellipse-shaped or egg-shaped equipotential lines not shown may be obtained. When attention is paid on one circle of the equipotential lines 13 and 14, any position on the one circle has the same potential. Although, in the figure, only four circles are depicted respectively in the positive electrode side and in the negative electrode side, in fact, an infinite number of equipotential lines continuously exist on the skin. Larger the circle is, lower the absolute value of its voltage is. In contrast, smaller the circle is, that is, nearer to the electrode the circle is, higher the absolute value of its voltage is. Herein, an electric field is a potential difference between two points divided by the distance between the two points. In this way, electric fields can be determined at other points on the skin, and thus the electric field distribution can be known.

Such an electric filed distribution can be known by an analysis using computer software commercially available. Figure 2 shows an example of an electric field analysis determined by computer analysis. This is an example of electric field distribution where needle-shaped electrodes having a diameter of 1 mm are used as a positive electrode and a negative electrode, a distance of 10 mm is provided between the positive and negative electrodes, and a voltage of 300 V are loaded between the electrodes. As apparent from the figure, it can be seen that the electric fields are distributed along a plane, or two-dimensionally.

Because the electric field strength is in correlation to the ability to form a hole, the skin of portions having strong electric field strength offers high permeability of materials, and the skin of portions having weak electric field strength offers low permeability. Therefore, for determining the structure of the electrode and device, it is very important to know that what degree of electric field is applied over what range, and what distribution is provided as a whole.

However, conventionally, a certain voltage is simply loaded and the load voltage is divided by a distance between the electrodes, and thus a value determined one-dimensionally is assumed to be an average electric field value over the whole skin. This gives no consideration to the two-dimensional distribution of electric fields as described above. For example, in the case where a voltage of 300 V is loaded over the distance of 1 cm between positive and negative electrodes, and in the case where a voltage of 600 V is loaded over the distance of 2 cm between positive and negative electrodes, both cases may be represented as having electric field strength of 300 V / cm determined one-dimensionally. However, in fact, the both cases are different in the electrode-electrode distances and the voltages, so that the two-dimensional distributions of electric fields over the skin are entirely different in the both cases and naturally, the skin permeabilities of a drug and the like are also different.

In this way, it is very important to determine an electrode structure and the output setting of an electroporation device (including a power supply device) after calculating two-dimensional electric field distributions for various electrodes.

Next, the present inventors have found that if the maximum electric field value (the maximum value of electric field strength) produced in the surface to which electroporation is applied is in the range of about 790 V / cm to 4000 V /cm, a target material can be effectively absorbed through skin or mucosa.

Also, the present inventors have found from electric field distributions that, if voltage-loaded area average strength of an electric field generated in the applied is in the range of about 7 V / cm to 730 V / cm, a target material can be effectively absorbed through skin or mucosa.

Herein, the voltage-loaded area average electric field strength refers to a average electric field value in the portion having electric fields equal to and higher than and 1 / 16 of the maximum electric field value. The following shows an example of how to obtain the voltage-loaded area average electric field strength. Figure 2 shows an electric field distribution measured by commercially available computer software (Volt S T, made by Photon Co. Lmt.). The measured electric field strength is classified into 16 levels from maximum value to 0 (level classification). In Figure 2, since the maximum value is 92356 V / m, electric fields are level classified for every 5772 V / m, obtained from 92356 / 16. For example, level 1 is in the range of 92356 to 86584 V /m, and in the same manner, level classification is performed from level 2 to level 14. Level 15 is in the range of 11545 to 5772 V / m, and level 16 is in the range of 5772 to 0 V / m. An intermediate value of each level is assumed to be an electric filed value of that level. Next, the ratio of the area occupied by each level to the area over which the electric field is loaded is determined (area ratio). In the example of Figure 2, the area ratios of each level are provided for the level 1 to 15 as follows, respectively; 0.000382831, 0.0004242, 0.000718383, 0.001512938, 0.002116709, 0.003256715, 0.004221758, 0.005088981, 0.00679246, 0.013719534, 0.020777283, 0.044858206, 0.127849139, 0.185078762, and 0.583202102. (Herein, level 16 is excluded from the calculation because its electric field is equal to and lower than 1 / 16 of the maximum value and includes 0 V / cm.) The area ratio and the level value of each level together are multiplied, and thus the sum of the resultant values is the voltage-loaded area average electric field strength. However, although the above example is measured using a computer, the measurement is not limited to this method provided that the distribution of electric fields and the maximum value of electric field strength can be determined, and the value obtained from those values is the voltage-loaded area average electric field strength.

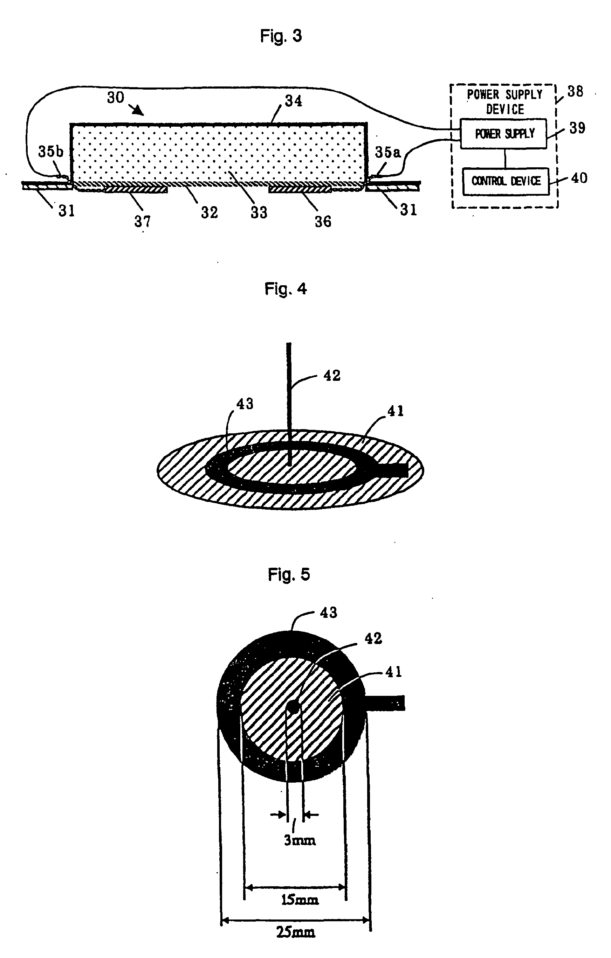

An electroporation device according to the invention is configured as shown in Figure 3, for example. That is, the present device comprises one pair or more pairs of electrodes consisting of at least a positive electrode and a negative electrode and / or a preparation 30 including a drug and the like, a power supply device 38 for supplying voltage and the like.

The preparation 30 comprises an adhesive layer 31 including a pressure sensitive adhesive for bringing the adhesive layer into contact with skin and mucosa, a dosed composition matter layer 33 including a drug, a backing film 34, a positive electrode 36, a negative electrode 37, electrode terminals 35a and 35b for connecting the positive electrode 36 and negative electrode 37 to the power supply device 38 respectively, and a discharge membrane 32 on which the electrodes are laminated. Also, the power supply device 38 comprises a power supply 39 for outputting voltage and a control device 40 for controlling the output of the power supply.

Although the materials of an electroporation electrode according to the invention are not particularly limited, carbon, platinum, gold, titanium, aluminum, nickel, iron, silver, silver chloride, copper, copper chloride, and an alloy of them may be used. Preferably, carbon, iron, and silver chloride are used because they can be easily molded by a printing technology.

Also, in assembling these electrodes into a preparation including a drug, as shown in Figure 3, the electrodes may be placed to make direct contact with skin or mucosa, or either one or both of the positive electrode and the negative electrode may be placed in proximity to skin or mucosa without direct contact therewith.

Herein, although the materials of the discharge membrane are not particularly limited, it is preferably selected as required according to a drug to be dosed (physiologically active substance) and the composition of the composition matter thereof. For example, when the composition matter and drugs are water-soluble, it is preferable to select a hydrophilic membrane such that the membrane permeation of the composition matter may not become rate-limiting. Conversely, when the composition matter and drugs are fat-soluble, it is preferable to select a hydrophobic membrane. Further, it is preferable to use a porous membrane having such pores as not to prevent permeation of dosed composition matter of a drug and the like. Herein, the pore sizes ranging preferably from 0.01 to 10 µm, more preferably from 0.1 to 5 µm, are suitable for the maintenance and permeability of a drug. Also, although the materials of the membrane are not particularly limited, it is possible to list porous membranes and foams of materials as described below and those obtained by subjecting these materials to chemical modification and treatment; nylon, polyvinylidene fluoride, cellulose, cellulose nitrate, polycarbonate, polysulfone, polyethylene, non-woven fabric, gauze, woven fabric, paper, absorbent cotton, polyethylene continuously foaming, polypropylene, vinyl acetate, polyolefin foam, polyamid foam, polyuretane.

Methods for laminating a electrode on the above discharge membrane include adhesion, printing, vapor deposition, plating and the like. Among these methods, printing is preferable because patterns and shape can be easily controlled by screen printing and the like. A lamination method by adhesion is a convenient and facile method and thus preferably used.

The electrodes laminated by such methods are preferably configured so as to have at least a pair of a positive electrode and a negative electrode provided on the membrane. Further, the distance between both the electrodes is determined by taking the above-described two-dimensional electric field into account.

Materials as the backing film are not particularly limited, provided that they are excellent in processing characteristics, flexibility and proper shape-retaining ability, and water-retaining characteristic. Herein, one kind or a mixture of more kinds of the macromolecular polymers as listed below and copolymers thereof are used, in which such materials are processed and molded into a film- like shape; for example, a chlorine-containing resin of a polymer of vinylidene chloride, vinyl chloride and the like, olefin base, ester base, styrene base, acrylic base, amide base, oxymethylene base, phenylene sulfide base, amide imide base, acrylonitril base, ether ketone, ether sulfone, sulfone ether imide, butadiene, and isoprene. Also, although the thickness of the film is not particularly limited, a thickness ranging from 5 to 250 µm is preferable, which is excellent in shape-retaining ability and flexibility.

The dosed composition matter may include, in addition to a primary drug, an electrolyte, an absorbefacient agent, a stabilizing agent, a pH adjustor, a bodying agent, a surface-active agent, an emulsifier, an ion-exchange resin, an ion-exchange membrane, non-woven fabric and the like, in consideration of absorbency and safety.

Also, a method of laminating the backing film and the discharge membrane is desirably the sealing performed by heat sealing and the like.

Although a drug used for the invention are not particularly limited, they may include a central analgesic, such as morphine, fentanyl, petidine, codeine, buprenorphine, butorphanol, eptazocine, and pentazocine; insulin, calcitonin, calcitonin-associated gene peptide, vasopressin, desmopressin, protirelin (TRH), adrenocorticotrophic hormone (ACTH), luteinizinghormone-releasing factor (LH-RH), growth hormone-releasing hormone (GRH), nerve growth factor (NGF), and other releasing factors; peptide group such as including angiotensin, parathyroid hormone (PTH), thyroid-stimulating hormone (TSH, thyrotropin), follicle-stimulating factor (FSH), luteinizing hormone (LH), prolactin, serumal gonadotrophic hormone, human chorionic gonadotrophin hormone (HCG), hypophyseal gonadotrophic hormone, growth hormone, somatostatin, somatomedin, glucagon, oxytocin, gastrin, secretin, endorphin, enkephalin, endothelin, cholecystokinin, neurotensin, interferon, interleukin, transferrin, erythropoietin (EPO), superoxide desmutase (SOD), granulocyte-colony sitimulating factor (G-CSF), intestinal vasodilator peptide (VIP), muramyl dipeptide, urogastrone, and human atrial natriuretic peptide (h-ANP); tranquilizer such as carbamazepine, chlorpromazine, diazopam, and nitrazepam; anti-malignant tumor agent such as phleomycin, adriamycin, 5-fluorouracil, and mitomycin; a cardiotonic drug such as digitalis, digoxin, and diitoxin; sex hormone such as estradiol and testosterone; and an antihypertensive agent such as reserpine and clonidine. Further, oligonucleotide, notably antisense DNA and triple chain-formable oligonucleotide, may be used.

A voltage supplied by the power supply device of the electroporation device can be specified by considering the structure of electrodes, that is, the electric field distribution which is influenced by the position relations between and the shapes, areas and the like of the positive electrode and the negative electrode. Also, power-applying patterns may include exponential and logarithmic waves or a rectangular wave, but the patterns are not limited to them.

### (Embodiments)

Hereinafter, the invention will be described by referring to embodiments, but the invention is not limited to them. (Embodiment 1) (determination of the maximum electric field value and an optimum value of voltage-loaded area average electric field strength)

### (Experiment of drug permeation)

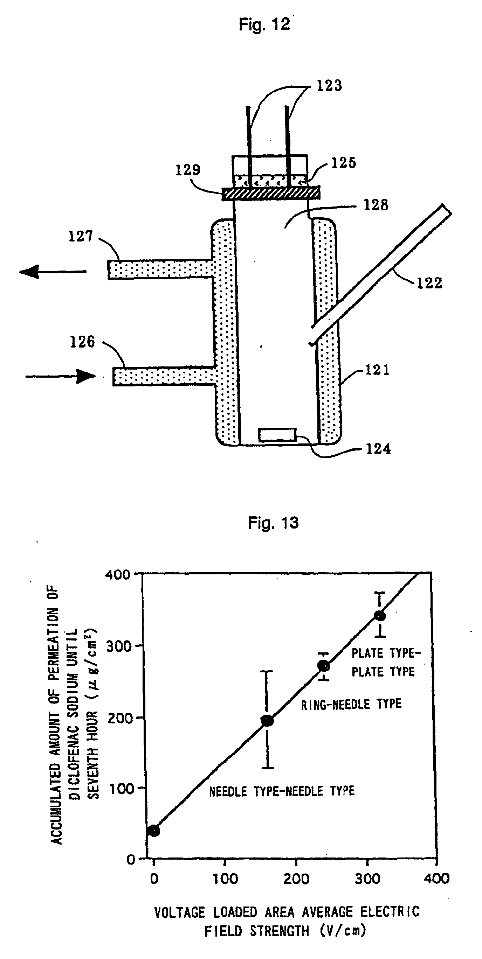

Figure 12 is a diagram showing an upright diffusion cell for permeation experiment. After anesthetizing a hairless rat having a weight of 220 g to 250 g with pentobarbital, a skin 129 of an abdominal part was removed therefrom and set to the upright diffusion cell for permeation experiment. Then, needle-shaped electrodes 123 of silver having a size of 1 mm were used as a positive electrode and a negative electrode, wherein the distance between the electrodes was set to 1, 5, 10, and 15 mm and 300 V was loaded between the electrodes. Also, in the same manner, the distance between the electrodes was set to 7.5 mm and 450 V or 600 V was loaded between the electrodes. The loading of voltage was performed ten times at a rate of once / minute, after that, paused for 50 minutes, again loaded ten times at a rate of once / minute, and paused for 50 minutes, and thus such a schedule was repeated over 8 hours. A sodium benzoate physiological active saline solution of 30 mg / ml was applied to a donor layer 125, and a receiver layer 128 was filled with physiological saline and stirred by a stirrer 124 during experiment. The receiver side solution was collected through a sampling port 122, and thus the concentration of sodium benzoate contained in the samples was measured by using high performance liquid chromatography to determine the amount of permeation. The permeation experiment was continued for 8 hours, and thus the maximum permeation rate during the period was determined. Further, reference numeral 121 in the figure denotes a jacket for keeping the receiver layer at a constant temperature of 37 °C, having a circulating structure in which hot water enters from an inlet 126 and goes out from an outlet 127.

### (Electric field analysis)

Commercially available software (Volt ST, made by Photon Co. Ltd.) was used to examine the electric field distribution in the skin under each condition of the electrode-to-electrode distances and the applied voltages which were used to perform the permeation experiment. The maximum value (the maximum value of electric field strength) of the electric field distribution obtained for each condition was determined and also the electric filed distributions were classified into 16 levels of electric field strength. An area of each of the levels from the highest level to 15th level was determined, and thus the area ratio of each level to the sum of the areas was determined. This technique was used to analyze all conditions (a total of 6) under which the permeation experiment was performed. Herein, this electric field analysis is associated with the case where the voltage was loaded once.

### (Comparison example 1)

As a comparison example 1, a permeation experiment was performed in the same manner as the embodiment 1. However, in this case, no voltage was loaded.

### (Result 1)

The results of the above experiment are shown in table 1.

**Table 1**

| Distance between electrodes: mm | Load voltage: V | Maximum value of electric field strength: V/cm | Voltage-loade d area average electric field strength: V/cm | Maximum permeation rate: µ mol/(cm²·h) | Other |
|---|---|---|---|---|---|
| - | - | - | - | 0.015 | Comparison example 1 |
| 1 | 300 | 17534 | 2946 | 0.016 | |
| 5 | 300 | 1430 | 254.0 | 0.032 | |
| 10 | 300 | 940 | 39.91 | 0.025 | |
| 15 | 300 | 791 | 7.3006 | 0.019 | |
| 7.5 | 450 | 2838 | 545.7 | 0.035 | |
| 7.5 | 600 | 3784 | 726.4 | 0.060 | |

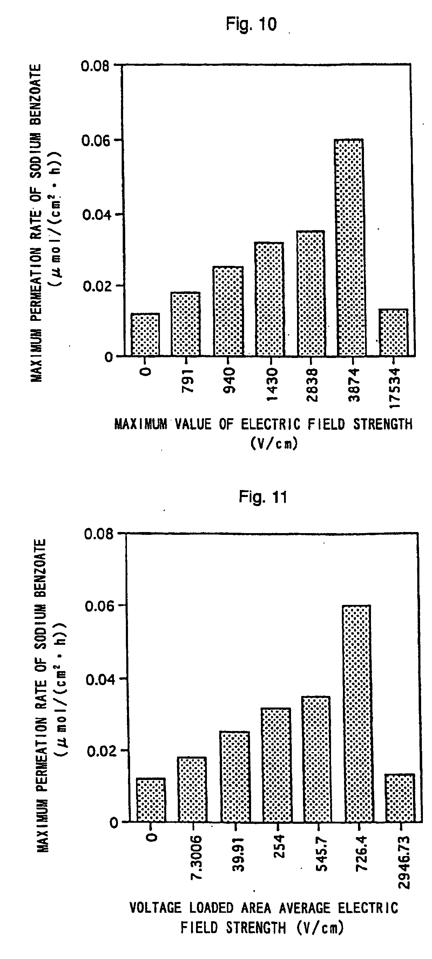

Figure 10 is a graph showing the relation between the maximum permeation rate of sodium benzoate and the maximum value of electric field strength. Also, Figure 11 is a graph showing the relation between the maximum permeation rate of sodium benzoate and the voltage loaded area average electric field strength. Therein, the case where the maximum value of electric field strength is 0 in Figure 10, and the case where the voltage-loaded area average electric field strength is 0 in Figure 11 show the results of the comparison example 1 in which no voltage was loaded. From the above results, it can be seen that if the maximum value of strength of an electric field generated by loading voltage at one time is in the range of about 790 V / cm to 4000 V / cm, the permeation of sodium benzoate is promoted as compared to the comparison example 1 in which no voltage was applied. On the other hand, when the maximum value of electric field strength exceeds about 4000 V / cm, the amount of permeation of sodium benzoate tends to be unchanged as compared to the case where no voltage was applied. Also, it can be seen that if the voltage-loaded area average electric filed strength produced by loading voltage at one time is in the range of about 7 to 730 V /cm, the permeation of sodium benzoate is promoted.

### (Embodiment 2)

By performing electric field analyses on the cases using three kinds of electrodes (preparations) having different structures, and making predictions about which electrode is most suitable, the structure was determined.

### (Experimental method)

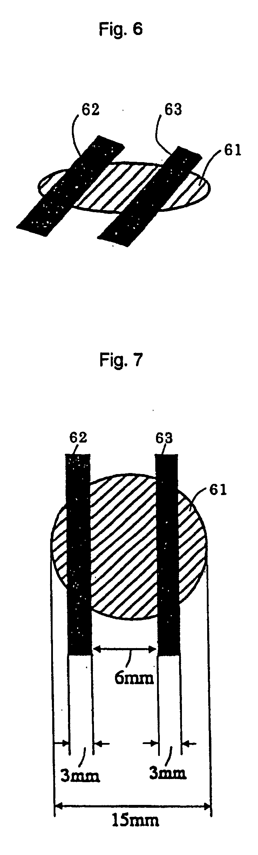

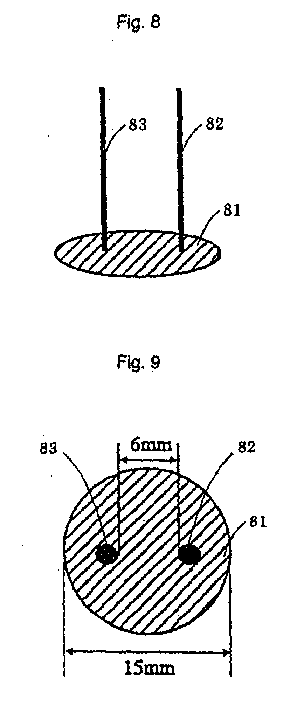

Figure 4 is a perspective diagram showing the example in which a ring type of positive electrode and a needle type of negative electrode (hereinafter, referred to as ring type-needle type) are applied to skin, and Figure 5 is a plan view of that example. Figure 6 is a perspective diagram showing the example where plate-type electrodes (plate type-plate type) of rectangular positive and negative electrodes are applied to skin, and Figure 7 is a plan view of that example. Figure 8 is a perspective diagram showing the example where needle types of positive electrode and negative electrode (needle type-needle type) are applied to skin, and Figure 9 is a plan view of that example. In each figure, reference numerals 41, 61, and 81 denote the skin, reference numerals 42, 62, and 82 denote the negative electrode, and reference numerals 43, 63, and 83 denote the positive electrodes. In the embodiment 2, these were subjected to the same test as those of the embodiment 1. Therein, the aqueous solution of diclofenac sodium of 20 mg / ml was used as the solution of a drug applied to the donor side, and a voltage of 200 V was loaded for one hour, at one time.

### (Result 2)

Since the above three kinds of electrode (ring type-needle type, plate type-plate type, needle type-needle type) all have an electrode-electrode distance of 6 mm, the conventional one-dimensional expression gives 200 V / 0.6 cm or 300 V / cm to all the three kinds. Therefore, it is predicted that all the electric fields have the same absorbency. However, if the electric fields are two-dimensionally analyzed according to the invention, the ring type-needle type has the voltage-loaded area average electric field strength of 242.4 V / cm, the plate type-plate type has that of 324.9 V / cm, and the needle type-needle type has that of 161.2 V / cm. Thus, the plate type-plate type has the highest voltage-loaded area average electric field strength. As demonstrated in the embodiment 1, if the voltage-loaded area average electric field strength is in the range of about 7 V / cm to 730 V / cm, the amount of permeation of a drug increases with increasing voltage-loaded area average electric field strength. From this fact, it is predicted that among the above three electrodes, the plate type-plate type may most efficiently permeate diclofenac sodium. Figure 13 is a graph showing the relation between the voltage-loaded area average electric field strength and the accumulated amount of permeation of diclofenac sodium until seventh hour. As apparent from this graph, as is predicted from the results of the analyses of the electric field distributions, the largest amount of permeation is obtained in the case of using the electrodes of the plate type-plate type, which offers the highest value of voltage-loaded area average electric field strength.

Although this embodiment shows the case where the electrodes having different structures load the same voltage, when electrodes having different structures load different voltages for example, it is possible to estimate which electrode may provide large amount of permeation. That is, the analysis according to the invention is effective not only for the structure of electrodes, but also for the setting of output voltages and the like. In this way, in the application of electroporation to transdermal or transmucosal, analysis using lines of electric force is performed, or the distribution of electric field intensity is analyzed and estimated in two-dimensional manner, and thereby, designing a device and an electrode structure for use in electroporation is very useful.

### (Embodiment 3) (electrodes designed and structure-determined based on the electric field analysis)

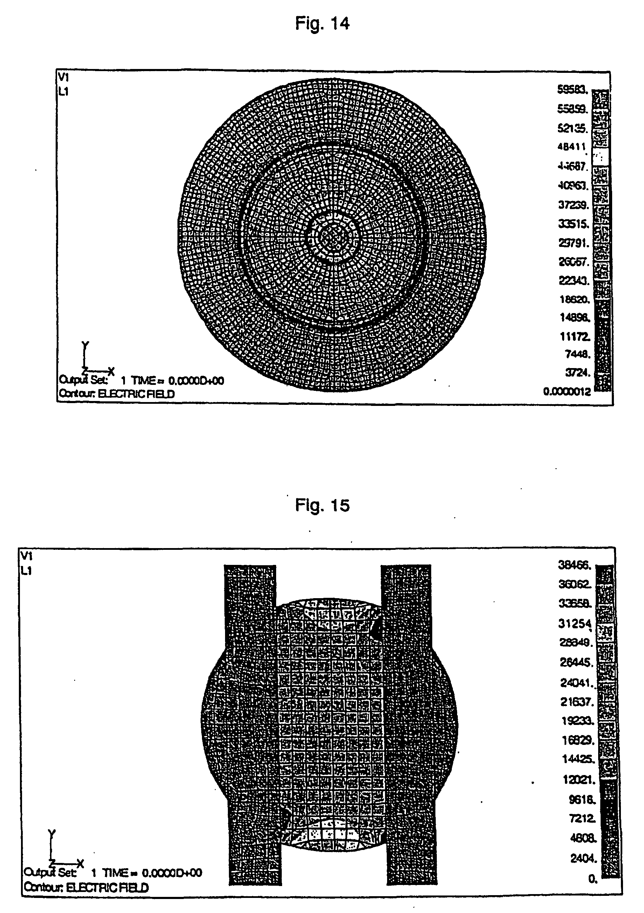

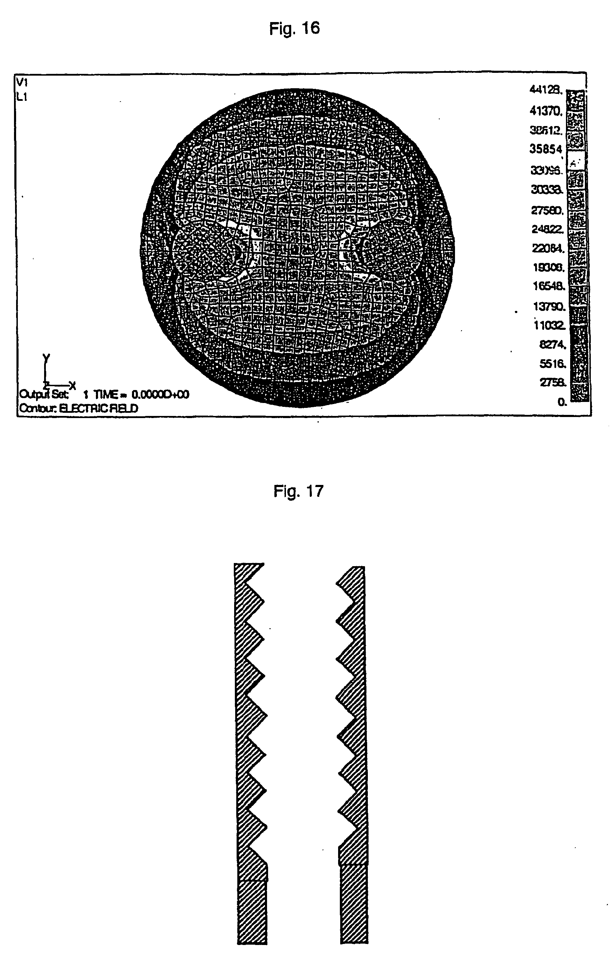

Figures 14 to 16 show the results of the electric field analyses performed in the embodiment 2. Herein, Figure 14 is a diagram showing the electric field distribution in the case of the ring type-needle type electrodes, analyzed by computer software, and Figure 15 similarly shows the electric field distribution in the case of the plate type-plate type electrodes, and Figure 16 similarly shows the electric field distribution in the case of the needle type-needle type electrodes. In the plate type-plate type that exhibits the most excellent permeability, because the electrode structures of the positive and negative electrodes have the same shape and the distance between the positive electrode and the negative electrode is fixed, the electric field are applied to the whole skin and high electric fields are relatively uniformly loaded, thereby providing high permeability. The electrodes and device is effectively designed such that the electric field intensity between the electrodes and in the voltage-loaded surface may be approximately uniform.

Also, as the results of analyzing the electric fields, it has been found that an electric field is not loaded directly underneath the electrodes, and that the electric field is highest in proximity of the electrodes, in which the absorbency of a drug also is excellent. Therefore, from the features of the structure and the features of the electric field distribution as described above, it is estimated that the structure of an electrode having higher absorbency satisfies a condition that the electrode is preferably small in area and long in length of its edge. Figures 17 to 19 show the shapes of the electrodes satisfying the conditions.

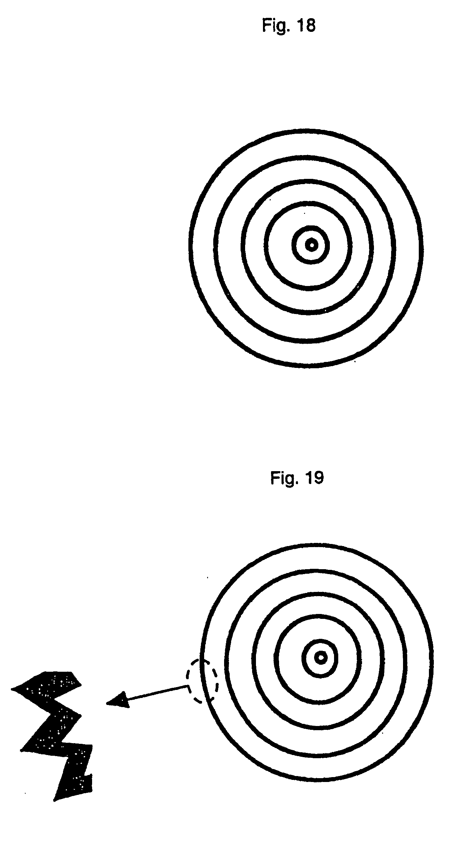

Figure 17 shows improved electrodes of a plate type-plate type. Herein, the area of the electrodes themselves is smaller because the electrodes have depths of cut in the shape of a triangle, and the edge has a longer peripheral length than the plate type because it has the shape of projections and depressions like a saw (opposing faces of both electrodes), and further the distance between the positive and negative electrodes is the same at any point. Also, Figure 18 shows an electrode concentrically spreading, in which positive electrodes and negative electrodes are alternately arranged and electric fields are substantially uniformly applied between the electrodes. Further, this has been improved into the electrode structure of Figure 19. These electrodes have triangle-shaped depths of cut provided in the edges thereof, providing a longer peripheral length of the whole electrodes, as a portion in a broken line is enlarged and shown in Figure 19. These depths of cut (the shape of projections and depressions) may be provided on the whole edges and may be provided on a portion of the edges. Further, the above structures are only exemplified, and the electrode structures of the embodiment are not limited to them.

### (Embodiment 4)

The permeation of sodium benzoate was studied, and the relation between the amount of permeation and electric fields loaded on skin was studied.

### (permeation experiment)

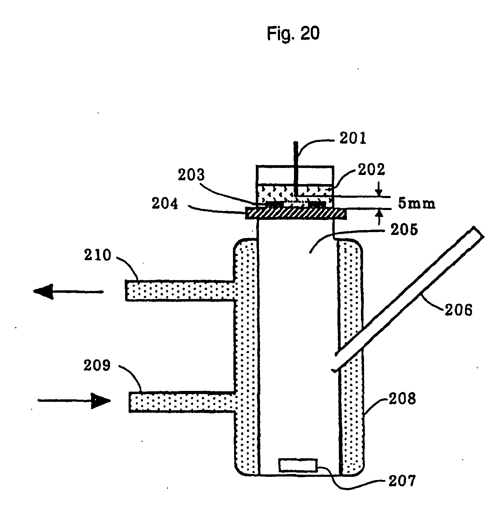

Figure 20 is a diagram showing an in vitro permeation experiment device and the situation of electrodes mounted therein. In this figure, reference numeral 201 denotes a needle type electrode made of silver (positive electrode), reference numeral 202 denotes a drug (aqueous solution of sodium benzoate of 30 mg / ml), reference numeral 203 denotes a ring type electrode made of silver (negative electrode, the same shape of the electrode 43), reference numeral 204 denotes skin (hairless rat-removed skin), reference numeral 205 denotes a receiver side solution (physiological saline), reference numeral 206 denotes a sampling port for collecting the receiver side solution, reference numeral 207 denotes a stirrer for stirring the receiver side solution, reference numeral 208 denotes a jacket for keeping the receiver layer at a constant temperature of 37 °C, having a circulating structure in which hot water enters from an inlet 209 and goes out from an outlet 210. In this device, although the negative electrode 203 makes contact with the skin, the positive electrode 201 does not make contact with the skin. A distance from the lower end of the positive electrode 201 to the skin 204 is 5 mm.

A gene pulser (Biollad) are used between the electrodes to provide electroporation once for one minute, loading 500 V over two hours, and the receiver side solution 205 is collected from the sampling port 206 over time, and then sodium benzoate contained in the sample is quantitatively tested with HPLC to determine the amount of permeation.

### (Electric field analysis)

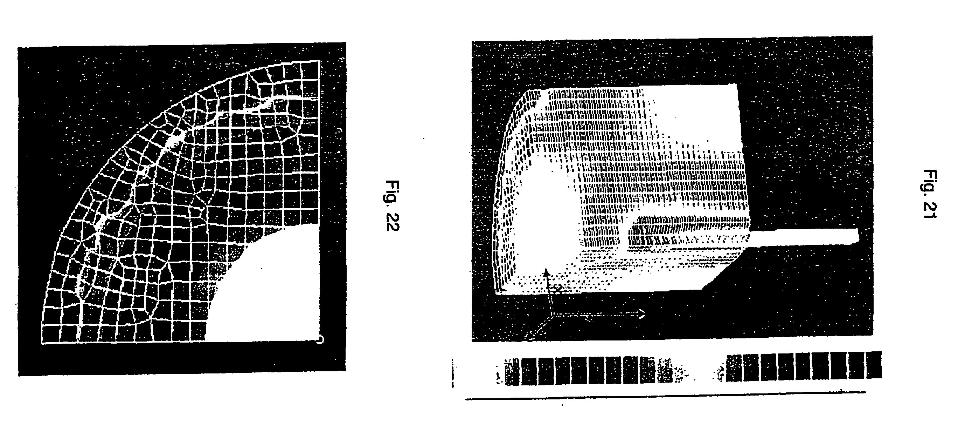

The conditions of the above permeation experiment (load voltage, position of the positive and negative electrodes and the distance between the electrodes, position of the skins) are input to the commercially available computer software described, thus determining the electric field distribution. In the embodiment 1, since the positive electrode, the negative electrode and the skin are positioned in the same plane, the electric field in the skin surface has been two-dimensionally determined. However, in this case, since the positive electrode, the negative electrode, and the skin are not positioned in the same plane, the distribution of the electric field has been determined by three-dimensional analysis. Figure 21 shows the results. Further, this electric field distribution determined in three dimensions allows determining of the two-dimensional distribution of electric fields in an arbitrary plane horizontal to the skin surface or a XY plane shown in Figure 21. Thus, the two-dimensional electric field distribution in the lowest XY plane (skin surface) of Figure 21 has been determined. The results are shown in Figure 22. This electric field distribution in the skin surface are used to perform the same electric field analysis as in the embodiment 1, thereby determining the voltage-loaded area average electric field.

### (Comparison example 2)

By using substantially the same device as in the embodiment 4, the same experiment was performed to determine the amount of permeation. However, no voltage was loaded.

### (Result 3)

In the comparison example 2, sodium benzoate of 5.01 µmol / cm² permeated until after the expiration of 8 hours. However, in the comparison example 2 in which no voltage was loaded, only 3.12 µmol / cm² permeated. This confirms that electroporation promotes the permeation of a drug.

On the other hand, the voltage-loaded area average electric field strength determined in the embodiment 4 is 357.16 V / cm, which is included in the above-mentioned range of about 7 V / cm to 730 V / cm.

That is, even if the positive electrode, the negative electrode, and the skin are not included in the same plane as in the present embodiment, electric field distribution is three-dimensionally examined and then the distribution of electric fields applied to the skin surface (or mucosa) is two-dimensionally analyzed, and as a result, when the voltage-loaded area average electric field strength is included in the above-described range, the effect of electroporation is obtained to promote the permeation of a drug.

### Industrial Applicability

The invention allows improving of the permeability of a drug in a device and electrode for electroporation, by specifying the maximum value of electric field strength and / or voltage-loaded area average electric field strength, and also by specifying the structure of electrodes and the like, as described above.

Further, two-dimensional or three-dimensional analyzing of electric field distribution allows providing a device and electrode for electroporation in which a load voltage is effectively available.

## Claims

1. Device for electroporation for applying electroporation to skin or mucosa, **characterized in that** for each one application of electroporation, the maximum value of the strength of an electric field generated in the surface to which electroporation is applied is set to be in the range of about 790 V / cm to 4000 V / cm.

2. Device for electroporation for applying electroporation to skin or mucosa, **characterized in that** for each one application of electroporation, the voltage-loaded area average strength of an electric field generated in the surface to which electroporation is applied is set to be in the range of about 7 V / cm to 730 V / cm.

3. Device for electroporation having a positive electrode and a negative electrode for applying electroporation to skin or mucosa, **characterized in that** the electroporation device is configured such that the strength of an electric field generated between the positive electrode and the negative electrode is substantially uniform.

4. Device for electroporation having a positive electrode and a negative electrode for applying electroporation to skin or mucosa, **characterized in that** by adjusting at least one of the distance between the positive electrode and the negative electrode and a load voltage, in one application of electroporation, the maximum value of the strength of an electric field generated in the surface to which electroporation is applied is set to be in the range of about 790 V / cm to 4000 V / cm, and / or the voltage-loaded area average electric field strength is set to be in the range of about 7 V / cm to 730 V / cm.

5. Device for electroporation according to Claim 4, **characterized in that** one of the positive electrode and the negative electrode is placed at a distance from the surface to which electroporation is applied.

6. Device for electroporation having a positive electrode and a negative electrode, **characterized in that** the electrode structures of the positive electrode and the negative electrode have the same shapes, and the distance between the positive electrode and the negative electrode is substantially constant.

7. Device for electroporation according to Claim 6, **characterized in that** the electrode structures of the positive electrode and the negative electrode are of a plate type.

8. Device for electroporation according to Claim 6, **characterized in that** the electrode structures of the positive electrode and the negative electrode are of a concentric circle-shaped ring type.

9. Device for electroporation according to any one of Claims 6 to 8, **characterized in that** the opposing planes of the positive electrode and the negative electrode have the shape of projections and depressions with respect to each other.

10. Electrode for electroporation comprising a positive electrode and a negative electrode, **characterized in that** the electrode structures of the positive electrode and the negative electrode have the same shapes, and the distance between the positive electrode and the negative electrode is substantially constant.

11. Electrode for electroporation according to Claim 10, **characterized in that** the electrode structures of the positive electrode and the negative electrode are of a plate type.

12. Electrode for electroporation according to Claim 10, **characterized in that** the electrode structures of the positive electrode and the negative electrode are of a concentric circle-shaped ring type.

13. Electrode for electroporation according to any one of Claims 10 to 12, **characterized in that** the opposing planes of the positive electrode and the negative electrode have the shape of projections and depressions with respect to each other.
